# FASCICULE DE BREVET EUROPEEN

(11) **EP 2 951 219 B1**
(45) Date de publication et mention de la délivrance du brevet: **23.11.2016**
(21) Numéro de dépôt: 14704858.1
(22) Date de dépôt: 29.01.2014
(51) Int. Cl.: C08F 220/56

(54) **NOUVEAUX POLYMERES PEIGNES UTILISABLES EN COSMÉTIQUE ET DETERGENCE**
NEUARTIGE KAMMPOLYMERE ZUR VERWENDUNG IN KOSMETIKARTIKELN UND REINIGUNGSMITTELN
NOVEL COMB POLYMERS THAT CAN BE USED IN COSMETICS AND IN DETERGENCY

(30) Priorité: 31.01.2013 FR 1350823
(43) Date de publication de la demande: 09.12.2015
(73) Titulaire: S.P.C.M. SA, 42160 Andrézieux Bouthéon (FR)
(72) Inventeur: BLONDEL, Frédéric, F-42600 Lezigneux (FR); SANNA, Antonin, F-42000 Saint Etienne (FR)
(74) Mandataire: Cabinet Laurent & Charras
(86) Numéro de dépôt international: PCT/FR2014/050158
(87) Numéro de publication internationale: WO 2014/118465

(56) Documents cités:
- DATABASE WPI Week 200957 Thomson Scientific, London, GB; AN 2009-L64412 XP002714566, & CN 101 475 691 A (UNIV CHENGDU TECHNOLOGY) 8 juillet 2009 (2009-07-08)

## Description

La présente invention concerne le secteur technique des polymères de structure "peigne", et plus précisément des polymères de structure peigne cationiques ayant des chaines pendantes hydrophiles. Ces polymères de structure peigne trouvent des applications, notamment dans les domaines de la cosmétique et de la détergence.

Un polymère de structure peigne présente une structure similaire à celle d'un peigne. En d'autres termes, il comporte une chaîne principale sur laquelle sont fixées des chaînes latérales qui peuvent être de nature différente et de longueur variable. Par exemple, ces chaînes latérales peuvent présenter des propriétés hydrophiles et/ou hydrophobes. Elles peuvent notamment être de type éthylène oxyde, propylène oxyde, alkyle, etc..., avec des longueurs de 2 à 500 motifs et de préférence de 5 à 200 motifs dans le cas d'une chaîne pendante de type poly (éthylène oxyde).

Les polymères de structure peigne de l'art antérieur comprennent notamment les polymères à base de motifs (méth)acrylate de polyéthylène glycol (PEGMA), et de motifs cationiques.

Le document EP372546 décrit des copolymères à base de PEGMA, de monomères de type (méth)acrylamides d'alkyles en C₁-C₈, et éventuellement des monomères cationiques.

Le document JP2002-322219 décrit des polymères contenant des motifs PEGMA en association avec des monomères hydrophobes à base de polypropylène glycol (PPO) ou de poly(oxyde de tétraméthylène), et des monomères cationiques.

Le document JP2003-055164 décrit des polymères réticulés contenant des motifs du type PEGMA; toutefois, ces polymères sont réticulés rendant ainsi le contrôle de leur synthèse plus délicat.

Le document JP2000-302649 décrit aussi une composition capillaire comprenant un polymère à base de monomères cationiques présentant des groupements amines quaternaires, de monomères à groupement polyéther, notamment de type PEG (polyéthylène glycol) ou PPO, et optionnellement de monomères hydrophobes (par exemple méthacrylate de stéaryle).

Le document JP07-285831 décrit des compositions capillaires contenant un polymère à base de monomères de type PEGMA associés à des monomères ioniques, cationiques ou amphotères, et des monomères additionnels de type (méth)acrylates d'alkyle en C₁-C₂₄, principalement hydrophobes.

Les documents EP1769011 et EP1765893 décrivent des polymères constitués majoritairement de motifs cationiques et de motifs PEGMA.

Le document WO2006/013268 décrit des polymères comprenant au moins un monomère du type (méth)acrylate de PEG associé à un monomère à caractère cationique (cationique, amphotère ou cationique et anionique).

Le document AU 2004 200 189 décrit un polymère pouvant comprendre un monomère du type acrylate de PEG associé à un monomère pouvant être cationique mais ne comprenant pas de fonction ammonium quaternaire.

Le document WO2006/013271 décrit une composition cosmétique comprenant un polymère contenant un monomère du type méthacrylate de PEG associé à un monomère ne comprenant pas de fonction ammonium quaternaire.

Comme déjà indiqué, les domaines d'application des polymères de structure peigne concernent notamment la cosmétique et la détergence. Ils peuvent donc être présents en tant qu'agent conditionneur, agent moussant ou agent d'aide à la déposition dans des compositions cosmétiques de produits corporels et capillaires, ou dans des détergents.

En solution aqueuse, les interactions entre un polyélectrolyte cationique et des espèces de charges opposées peuvent se traduire par la précipitation d'un complexe. Ce complexe (ou coacervat) correspond à l'adsorption des espèces anioniques sur le polyélectrolyte. Dans certains cas, le complexe peut être solubilisé en ajoutant un excès d'espèces anioniques.

Il est connu que ces coacervats présentent des propriétés intéressantes de conditionnement et d'aide à la déposition d'actif (*Principles of polymer science and technology in cosmetics and personal care - Polymer*/*surfactant interaction in applied systems E.D Doddard*).

Toutefois les polymères communément utilisés comportent un certain nombre d'inconvénients.

Par exemples, les polymères d'origine naturelle tels que les gommes guars ou les hydroxycellulose, ne possèdent pas naturellement de charges cationiques. Par conséquent, une étape supplémentaire est donc nécessaire pour les rendre cationique. Or, la répartition des charges sur le polymère lors de cette étape est aléatoire, ce qui ne garantit pas la disponibilité de la charge qui peut se trouver aussi bien sur la chaîne principale que sur les chaînes latérales.

Ces polymères ont aussi comme inconvénient leur mise en oeuvre. Du fait de leur origine naturelle la mise en solution est longue et fastidieuse.

En outre, les polymères naturels, et en particulier les dérivés de guar, sont aussi utiles dans d'autres applications, comme l'industrie alimentaire ou le textile, mais aussi l'exploitation des ressources d'huile et de gaz non conventionnels. Ceci crée une pression sur la disponibilité de ces produits et engendre des problèmes de prix.

Dans un domaine technique tout à fait différent qui est celui de l'EOR et dans lequel la question de la formation des coacervats ne se pose pas, le document CN 101 475 691 A décrit un microgel contenant un polymère réticulé obtenu à partir d'un monomère cationique, d'un monomère anionique et d'un monomère fonctionnel. L'exemple 5 décrit un polymère formé de 58% d'acrylamide, de 7% de DADMAC et de 35% de lauryl polyoxyethylène ether acrylate.

Le problème que se propose de résoudre la Demanderesse est de fournir des polymères cationiques pouvant former des coacervats lors de leur dilution en présence d'espèces anioniques, tout en s'affranchissant des contraintes de l'art antérieur.

La Demanderesse a mis au point des polymères permettant notamment d'optimiser la formation de ces coacervats.

La présente invention concerne ainsi des polymères qui, une fois incorporés dans une composition cosmétique ou détergente, facilitent la formation de coacervats résultant de l'attraction ionique entre deux composés de charges opposées.

Les polymères ont ainsi une meilleure interaction avec les composés pouvant être présents dans des formulations comme les surfactants, d'autres polymères et des principes actifs. Ces polymères ont aussi une meilleure affinité avec le cheveu et la peau pour la cosmétique ou les surfaces pour les détergents.

La présente invention a donc pour objet un copolymère hydrosoluble à motifs éthyléniques, comprenant, en pourcentage en masse par rapport à la masse totale du copolymère :
- 1 à 40% d'au moins un monomère cationique dont la cationicité provient exclusivement de fonction(s) ammonium quaternaire ;
- 59.99 à 98% d'au moins un monomère non ionique ;
- 0,01 à 10% d'au moins un monomère de formule (I) ; 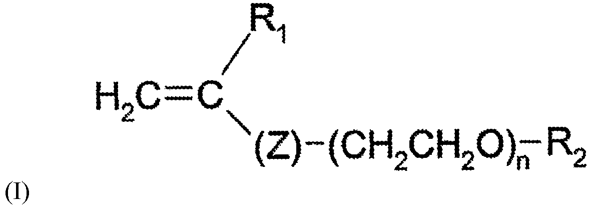 dans laquelle :
   - R₁ est un atome d'hydrogène ou un radical méthyle ;
   - Z est une fonction choisie dans le groupe comprenant une liaison covalente, un atome d'oxygène, le groupement divalent -CH₂ - O-, -C(=O)-O-, et -C(=O)-NH- ;
   - n est un nombre entier compris entre 2 et 200 ;
   - R₂ est un atome d'hydrogène ou un radical carboné, saturé ou insaturé, éventuellement aromatique, linéaire, ramifié ou cyclique, comprenant 1 à 30 atomes de carbone, comprenant de 0 à 4 hétéroatomes choisis dans le groupe comprenant O, N, et S.

De manière avantageuse, n est compris entre 7 et 45.

De manière avantageuse, le copolymère hydrosoluble à motifs éthyléniques, comprend, en pourcentage en masse par rapport à la masse totale du copolymère :
- 1 à 30%, avantageusement entre 22.5 et 30% d'au moins un monomère cationique dont la cationicité provient exclusivement de fonction(s) ammonium quaternaire ;
- 60 à 90% avantageusement entre 60 et 80% de préférence entre 60 et 70% d'au moins un monomère non ionique ;
- 0,01 à 10% d'au moins un monomère de formule (I)

Par le terme « hydrosoluble », on désigne un copolymère pouvant être mis en solution aqueuse, à hauteur d'au moins 50 g/L à 25°C, sans laisser subsister de particules insolubles.

En outre, selon une autre caractéristique de l'invention, la cationicité du copolymère est avantageusement comprise entre 0,05 et 3 meq/g, préférentiellement entre 0.05 et 1.8 meq/g.

La cationicité ou densité de charge cationique correspond au nombre d'équivalent cationique par unité de masse.

En d'autres termes, dans le cas où le copolymère comprend un monomère cationique A, et un monomère non cationique B, elle est déterminée selon la formule suivante : Cationicité (meq/g) = (1000 ₓ %A) / (%A ₓ Mw*_{A}* + %B ₓ Mw*_{B}*)
dans laquelle :
- %A représente le pourcentage molaire du monomère cationique A ;
- %B représente le pourcentage molaire du monomère non cationique B ;
- Mw*_{A}* représente la masse molaire du monomère cationique A ;
- Mw*_{B}* représente la masse molaire du monomère non cationique B.

La densité de charge cationique dépend donc des proportions de monomères ainsi que de leurs masses molaires respectives. Par conséquent, à ratio de monomères équivalents, deux polymères ne présenteront pas nécessairement la même densité de charge cationique eue égard à la masse molaire de chacun des monomères.

Selon un mode de réalisation préféré, dans le monomère de formule (I), R₂ est au choix un atome d'hydrogène; un radical benzyle; un radical phényle éventuellement substitué par au moins un alkyle en C₁-C₁₂, un radical alkyle en C₁-C₃₀ linéaire ou ramifié, comprenant éventuellement au moins un groupement cyclique, et éventuellement au moins un groupement aromatique, notamment en C₁-C₂₂, voire en C₂-C₁₆, comprenant éventuellement 1 à 4 hétéroatomes choisis parmi O, N et S. On peut notamment citer les radicaux méthyle, éthyle, propyle, benzyle, éthylhexyle, lauryle, stéaryle, béhényle.

Les monomères de formule (I) tout particulièrement préférés peuvent être choisis dans le groupe comprenant les (méth)acrylates de poly(éthylène glycol), les (méth)acrylates de méthyl-poly(éthylène glycol), les alkylènes de vinyle de poly(éthylène glycol), les butylène d'oxyvinyle de poly(éthylène glycol). De préférence, il s'agit de monomères dont la masse molaire est avantageusement comprise entre 80 et 8000 g/mol, plus avantageusement entre 300 et 2000 g/mol.

Parmi les monomères de formule (I) préférés, on peut citer :
- le (méth)acrylate de poly(éthylène glycol) dans lequel R₁ = H ou CH₃ ; Z = C(=O)-O- ; R₂ = H ; avec n = 2 à 200 ;
- le (méth)acrylate de méthyl-poly(éthylène glycol), aussi appelé (méth)acrylate de méthoxy-poly(éthylèneglycol), dans lequel R₁ = H ou CH₃ ; Z = C(=O)-O- ; et R₂=CH₃; avec n = 2 à 200;
- les (méth)acrylates d'alkyl-poly(éthylène glycol) dans lesquels R₁ = H ou CH₃ ; Z = C(=O)-O- ; et R₂ = alkyl en C₁-C₃₀ ; avec n = 2 à 200 ;
- les (méth)acrylates de phényl-poly(éthylène glycol), aussi appelés (méth)acrylate de poly(éthylène glycol) phényl éther, dans lesquels R₁ = H ou CH₃ ; Z = C(=O)-O- ; et R₂ = phényle ; avec n = 2 à 200.
- les alkylènes de vinyle de poly(éthylène glycol), dans lesquels R₁ = H ou CH₃ ; Z = -CH2-O- ; R₂ = H ou alkyl en C₁-C₃₀ ; avec n = 2 à 200 ;
- les butylène d'oxyvinyle de poly(éthylène glycol), dans lesquels R₁ = H ou CH₃ ; Z = liaison covalente ; R₂ = H ou alkyl en C₁-C₃₀ ; avec n = 2 à 200 ;

Parmi les monomères commerciaux, on peut citer :
- les méthacrylates de polyéthylène glycol 8000 ou 4000 commercialisés par Monomer & Polymère Dajac laboratories ;
- les méthacrylates de poly(éthylène glycol), de masse molaire 5000 g/mol, disponibles chez EVONIK sous la dénomination commerciale VISIOMER^{®} ;
- les méthacrylates de hydroxy-poly(éthylène glycol) commercialisés par CLARIANT sous la dénomination commerciale POLYGLYKOL^{®} MA
- les alkylènes de vinyle de poly(éthylène glycol) commercialisés par Liaoning oxiranchem company sous la dénomination commerciale OXAB ;
- les butylène d'oxyvinyle de poly(éthylène glycol) commercialisés par Zhejiang Huangma Chemical Industry group sous la dénomination commerciale HMXB-45.

Le ou les monomères cationiques pouvant être utilisés dans le cadre de l'invention peuvent être choisis, notamment parmi les monomères du type acrylamide, acrylique, vinylique, allylique ou maléique possédant une fonction ammonium quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de methacrylamido propyltrimethyl ammonium (MAPTAC).

Le ou les monomères non ioniques pouvant être utilisés dans le cadre de l'invention peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide et le N-méthylolacrylamide. Egalement, peuvent être utilisés la N-vinylformamide, le N-vinyl acetamide, la N-vinylpyridine et la N-vinylpyrrolidone, l'acryloyl morpholine (ACMO) et la diacétone acrylamide. Un monomère non ionique préféré est l'acrylamide.

Selon certains modes de réalisation, en plus des monomères ci-dessus, le(s) copolymères hydrosolubles peuvent également comprendre un ou plusieurs monomères hydrophobes choisis, notamment, parmi les monomère de type acrylamide, acrylique, vinylique, allylique ou maléique possédant une fonction hydrophobe pendante choisis préférentiellement parmi les dérivés de l'acrylamide comme les N-alkylacrylamides, par exemple, le diacétone acrylamide, le N-tert-butylacrylamide, l'octylacrylamide, et les N,N-dialkylacrylamides comme le N,N-dihexylacrylamide et les dérivés d'acide acrylique comme les alkyl acrylates et méthacrylates. Egalement peuvent être utilisés les dérivés de monomères vinyliques comme la N-vinylformamide, le N-vinyl acétamide, la N-vinylpyridine, et le N-vinylimidazole. Le copolymère peut en outre être structuré par au moins un agent de structure, pouvant être choisi dans le groupe comprenant des monomères à insaturation polyéthylénique (ayant au minimum deux fonctions insaturées), comme par exemples les fonctions vinyliques, allyliques, acryliques et époxy et l'on peut citer par exemple le méthylène bis acrylamide (MBA), la triallyamine, le diacrylate de polyéthylène glycol, ou encore par des macroamorceurs tels que les polyperoxydes, polyazoiques et les polyagents de transfert tels que les polymères polymercaptants.

Selon l'invention, et de manière avantageuse, le polymère n'est pas réticulé. Il peut être linéaire ou structuré, c'est à dire branché, star (en forme d'étoile) ou comb (en forme de peigne).

Quelle que soit sa structure, le polymère objet de l'invention présente des chaînes pendantes hydrophiles issues du monomère de formule (I).

De manière générale, les polymères de l'invention ne nécessitent pas de développement de procédé de polymérisation particulier. En effet, ils peuvent être obtenus selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; ou de polymérisation micellaire.

Le polymère peut se présenter sous forme liquide ou solide lorsque sa préparation inclut une étape de séchage tel que le spray drying, le séchage sur tambour ou encore le séchage micro-ondes.

Par le terme « espèces anioniques », on entend tous les éléments macromoléculaires, ayant un caractère anionique, couramment présents dans les formulations de type cosmétique, détergent ou autre.

Par conséquent, la présente invention concerne également l'utilisation du copolymère décrit ci-avant dans une formulation cosmétique ou détergente.

De manière non limitative, ces espèces ioniques peuvent être :
(i) Les tensioactifs anioniques parmi lesquels on peut citer, seuls ou mélangés, les sels (en particulier les sels de métaux alcalins, notamment les sels de sodium, les sels d'ammonium, les sels d'aminés, les sels d'aminoalcools ou les sels de magnésium) des composés suivants : les alkylsulfates, les alkyléthersulfates, les alkylamidoéthersulfates, les alkylarylpolyéthersulfates, les monoglycérides sulfates, les alkylsulfonates, les alkylphosphates, les alkylamidesulfonates, les alkylarylsulfonates, les alpha-oléfine-sulfonates, les paraffinesulfonates, les alkylsulfosuccinates, les alkyléthersulfosuccinates, les alkylamide- sulfosuccinates, les alkylsulfosuccinamates; les alkylsulfoacétates, les alkylétherphosphates, les acylsarcosinates, les acyliséthionates, et les N-acyltaurates. Le radical alkyle ou acyle de tous ces différents composés comporte de préférence de 8 à 24 atomes de carbone, alors que le radical aryle désigne de préférence un groupement phényle ou benzyle.
   On peut également citer les sels d'acides gras tels que les sels des acides oléique, ricinoléique, palmitique, stéarique, les sels d'acides d'huile de coprah ou d'huile de coprah hydrogénée; les sels d'acyl-lactylates dont le radical acyle comporte 8 à 20 atomes de carbone; les sels d'acides d'alkyl D galactoside uroniques ainsi que les sels d'acides alkyl (C₆-C₂₄) éther carboxyliques polyoxyalkylénés, les sels d'acides alkyl(C₆-C₂₄)aryl éther carboxyliques polyoxyalkylénés, les sels d'acides alkyl(C₆-C₂₄) amido éther carboxyliques polyoxyalkylénés, en particulier ceux comportant de 2 à 50 groupements oxyde d'éthylène, et leurs mélanges.
(ii) Les polyelectrolytes anioniques comprenant au moins un monomère présentant une fonctionnalité acrylique, vinylique, maléique, fumarique, ou allylique et contenant un groupe carboxy, phosphonate, sulfonate, ou un autre groupe à charge anionique. Il peut notamment s'agir de : l'acide acrylique, l'acide méthacrylique, l'acide itaconique, l'acide crotonique, l'acide maléique, l'acide fumarique, et les monomères de type acide fort présentant par exemple une fonction de type acide sulfonique ou acide phosphonique tels que l'acide 2-acrylamido-2-méthylpropane sulfonique, l'acide vinylsulfonique, l'acide vinylphosphonique, l'acide allylsulfonique, l'acide allylphosphonique.
(iii) Les polymères naturels ayant un caractère anionique, pouvant être choisis dans le groupe comprenant les polysaccharides tels que la cellulose, l'amidon, la gomme de guar, la gomme de guar hémicellulose, la gomme arabique, le glucomannane, la gomme de caroube, le pullulane, le curdane, la gomme de xanthane, la gomme de gellane, la gomme de Carraghénane, la gomme de dextrane, la gomme adragante, la gomme de welane, la gomme rhamsane, l'acide hyaluronique, l'inuline, la pectine, la lignine, la chitine, l'alginate, l'agar-agar ou leurs dérivés.

L'invention a également pour objet un procédé permettant d'optimiser la formation de coacervats dans une composition cosmétique ou détergente consistant à incorporer dans ladite composition le polymère précédemment décrit.

L'invention et les avantages qui en découlent ressortiront mieux des figures et exemples suivants donnés afin d'illustrer l'invention et non de manière limitative.

### FIGURES

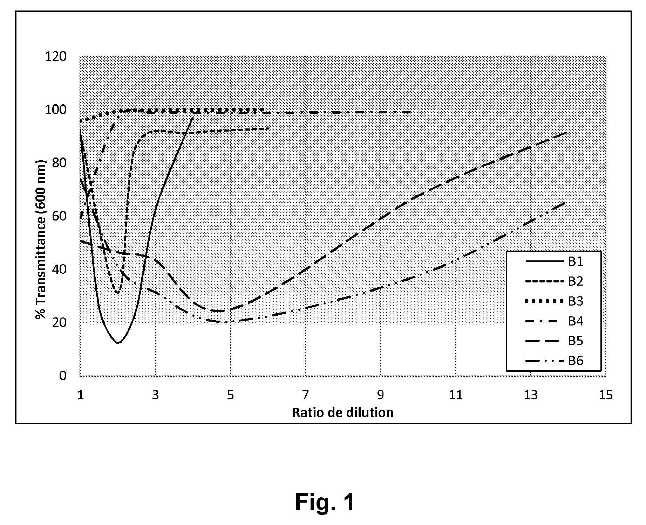

La figure 1 représente le graphe correspondant à la transmittance de solutions contenant un polymère cationique et des espèces anioniques, en fonction de la dilution de ladite solution.

### EXEMPLES DE REALISATION DE L'INVENTION

### A/ Préparation des copolymères :

### Préparation du polymère A1 : polymère selon l'invention.

Dans un réacteur équipé d'un système d'agitation mécanique, d'un condenseur, d'un thermomètre et d'une arrivée d'azote, on charge :
- 19,7 g (27% massique) d'une solution à 64% de chlorure de diméthyldiallylammonium
- 62,7 g (68% massique) d'une solution à 50% d'acrylamide
- 2,1 g (5% massique) de mPEG 2000 MA (Visiomer mPEG 2005 MA, Evonik). Il s'agit d'un méthacrylate de methoxypolyéthylèneglycol dont la masse molaire est de 2000 g/mol.
- 365,5 g d'eau

Le milieu réactionnel est désoxygéné avec un flux d'azote, et chauffé à 75°C.

Séparément, on prépare une solution d'initiateur en introduisant 0,45 g de 2,2' azobis(2-amidinopropane) dihydrochloride (V50, Wako) dans 20 g d'eau.

Quand la température du milieu a atteint 75°C, on démarre l'ajout progressif de la solution d'initiateur. La solution est ajoutée pendant 120 minutes, puis le milieu est maintenu à 75°C pendant 120 minutes supplémentaires pour compléter la polymérisation.

On laisse le mélange revenir à température ambiante, puis on ajuste le pH entre 4 et 6 en utilisant une solution aqueuse de NaOH ou d'acide citrique à 50% en masse.

Le produit obtenu est une solution liquide dont la concentration en polymère est de 10% en masse par rapport à la masse de la solution. La solution présente une viscosité de 3000 cps (Brookfield LVT, module 3, 30 rpm). Le polymère A1 possède une densité de charge cationique de 1,7 meq/g.

### Préparation du polymère A2 : polymère ramifié selon l'invention.

Le polymère A2 est préparé en suivant les conditions expérimentales décrites lors de la préparation de A1, mais en ajoutant 88 mg de diacrylate de polyethylene glycol 600 lors de la charge du réacteur en lieu et place du méthacrylate de methoxypo lyéthylèneglycol.

Le produit obtenu est une solution liquide dont la concentration en polymère est de 10% en masse par rapport à la masse de la solution. La solution présente une viscosité de 14000 cps (Brookfield LVT, module 4, 30 rpm).

Le polymère A2 présente une structure ramifiée. Le polymère A2 possède une densité de charge cationique de 1,7 meq/g.

### Préparation du polymère A3 : modification de la composition du polymère (contre-exemple).

Le polymère A3 est préparé en suivant les conditions expérimentales décrites lors de la préparation de A1 mais en chargeant les quantités suivantes dans le réacteur :
- 17,7 g (22.5% massique) d'une solution à 64% de chlorure de diméthyldiallylammonium
- 56,1 g (55% massique) d'une solution à 50% d'acrylamide
- 11,2 g (22.5% massique) de mPEG 2000 MA (Visiomer mPEG 2005 MA, Evonik). Il s'agit d'un méthacrylate de methoxypolyéthylèneglycol dont la masse molaire est de 2000 g/mol.
- 365 g d'eau

Le produit obtenu est une solution liquide dont la concentration en polymère est de 10% en masse_par rapport à la masse de la solution. La solution présente une viscosité de 800 cps (Brookfield LVT, module 3, 30 rpm). Le polymère A3 possède une densité de charge cationique de 1,5 meq/g.

### B/ Etude de la formation de coacervat entre les polymères et des polyéléctrolytes anioniques

### Méthode de caractérisation

L'objectif est de mettre en évidence la capacité d'un polymère à former un coacervat avec des espèces anioniques lors de la dilution.

Les formulations B1-B6 du tableau 1 sont préparées à partir des polymères A1-A3 et des polymères de l'art antérieur.

**Tableau 1 : Formulations B1-B6 (pourcentages en poids).**

| Formule | B1 | B2 | B3 | B4 | B5 | B6 |
|---|---|---|---|---|---|---|
| Eau | qsp 100% | qsp 100% | qsp 100% | qsp 100% | qsp 100% | qsp 100% |
| Polymère A1 | 1% | | | | | |
| Polymère A2 | | 1% | | | | |
| Polymère A3 | | | 1% | | | |
| Polyquaternium 7 (Flocare C107) | | | | 1% | | |
| Guar hydroxypropyltrimo nium chloride (Jaguar C13S) | | | | | 1% | |
| Guar hydroxypropyltrimo nium chloride (Jaguar Excel) | | | | | | 1% |
| Cocamidopropyl betaine | 3% | 3% | 3% | 3% | 3% | 3% |
| Sodium Lauryl Ether Sulfate | 8% | 8% | 8% | 8% | 8% | 8% |
| NaCl | 1% | 1% | 1% | 1% | 1% | 1% |

La mise en oeuvre des guars cationiques (formulation B5 et B6) est longue et délicate. Leur mise en solution dans l'eau nécessite une agitation élevée pendant une durée de 20 minutes environ (documents techniques Rhodia : *Recommended procedure for formulating cationic Jaguar, Jaguar Excel data sheet* et *Jaguar C13S data sheet).*

Les polymères préparés selon les exemples A1, A2 et A3 présentent l'avantage d'être parfaitement solubilisés dans l'eau après quelques secondes d'agitation uniquement.

Les formulations B1 à B6 sont ensuite diluées dans l'eau suivant plusieurs ratios. La transmittance à 600 nm de chacune des solutions ainsi obtenues est mesurée à l'aide d'un spectrophotomètre.

La formation d'un coacervat lors de la dilution est révélée par une baisse de la transmittance (apparition d'un trouble) (tableaux 2 et 3 ; figure 1).

### Résultats :

Les dilutions des formulations B1 à B6 conduisent aux mesures de transmittance suivantes :

**Tableau 2 : Transmittance mesurée pour des solutions diluées, à base des formulations B1-B3.**

| B1 | | B2 | | B3 | |
|---|---|---|---|---|---|
| Ratio de dilution | % Transmittance (600 nm) | Ratio de dilution | % Transmittance (600 nm) | Ratio de dilution | % Transmittance (600 nm) |
| 1 | 92,4 | 1 | 91 | 1 | 95,7 |
| 1,5 | 26,1 | 2 | 31,2 | 2 | 99,4 |
| 2 | 12,3 | 2,5 | 86,4 | 3 | 99,8 |
| 2,5 | 24,7 | 4 | 91,2 | 6 | 100 |
| 3 | 62 | 6 | 92,9 | | |
| 4 | 96,9 | | | | |

**Tableau 3 : Transmittance mesurée pour des solutions diluées, à base des formulations B4-B6.**

| B4 | | B5 | | B6 | |
|---|---|---|---|---|---|
| Ratio de dilution | % Transmittance (600 nm) | Ratio de dilution | % Transmittance (600 nm) | Ratio de dilution | % Transmittance (600 nm) |
| 1 | 59,3 | 1 | 50,6 | 1 | 74 |
| 2 | 96,7 | 2 | 46,3 | 2 | 41,3 |
| 3 | 98,9 | 3 | 43,3 | 3 | 31,3 |
| 5 | 98,8 | 5 | 24,8 | 5 | 20,4 |
| 10 | 99,2 | 10 | 67,4 | 10 | 37,6 |
| | | 14 | 91,6 | 14 | 65,3 |

Les dilutions des formulations B1 à B6 sont illustrés par la figure 1.

### Conclusion :

Lorsqu'ils sont formulés avec des espèces anioniques puis dilués, les polymères de l'invention permettent la formation de coacervats.

La transmittance des formulations B1-B6 est comparée à la transmittance référence de l'eau (100%).

## Revendications

1. Copolymère hydrosoluble comprenant, en masse par rapport à la masse totale du copolymère :
- 1 à 40% d'au moins un monomère cationique dont la cationicité provient exclusivement de fonction(s) ammonium quaternaire ;
- 59.99 à 98% d'au moins un monomère non ionique ;
- 0,01 à 10% d'au moins un monomère de formule (I) ; 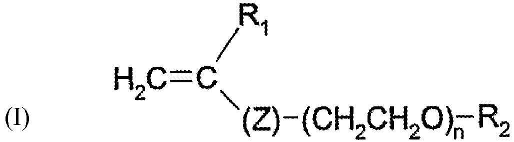 dans laquelle :
- R₁ est un atome d'hydrogène ou un radical méthyle ;
- Z représente une fonction choisie dans le groupe comprenant une liaison covalente, un atome d'oxygène, le groupement divalent -CH₂- O-,-C(=O)-O-, et -C(=O)-NH-,;
- n est un nombre entier compris entre 2 et 200 ;
- R₂ est un atome d'hydrogène ou un radical carboné, saturé ou insaturé, éventuellement aromatique, linéaire, ramifié ou cyclique, comprenant de 1 à 30 atomes de carbone, et de 0 à 4 hétéroatomes choisis dans le groupe comprenant O, N, et S ;
le copolymère présentant une densité de charge cationique comprise entre 0,05 et 3 meq/g.

2. Copolymère selon la revendication 1, ***caractérisé* en ce que** le copolymère comprend en masse par rapport à la masse totale du copolymère :
- 1 à 30%, avantageusement entre 22.5 et 30% d'au moins un monomère cationique dont la cationicité provient exclusivement de fonction(s) ammonium quaternaire ;
- 60 à 90% avantageusement entre 60 et 80% de préférence entre 60 et 70% d'au moins un monomère non ionique ;
- 0,01 à 10% d'au moins un monomère de formule (I) ;

3. Copolymère selon la revendication 1, ***caractérisé* en ce que** le copolymère présente une densité de charge cationique comprise entre 0,05 et 1,8 meq/g.

4. Copolymère selon l'une des revendications précédentes, ***caractérisé* en ce que** le monomère de formule (I) est choisi dans le groupe comprenant :
- le (méth)acrylate de poly(éthylène glycol) dans lequel R₁ = H ou CH₃ ; Z = C(=O)-O ; R₂ = H; n = 2 à 200;
- le (méth)acrylate de méthyl-poly(éthylène glycol), dans lequel R₁ = H ou CH₃ ; Z = C(=O)-O- ; R₂ = CH₃ ; n = 2 à 200 ;
- les (méth)acrylates d'alkyl-poly(éthylène glycol) dans lesquels R₁ = H ou CH₃ ; Z = C(=O)-O ; R₂ = alkyl en C₁-C₃₀ ; n = 2 à 200 ;
- les (méth)acrylates de phényl-poly(éthylène glycol), dans lesquels R₁ = H ou CH₃ ; Z = C(=O)-O ; R₂ = phényle ; n = 2 à 200.
- les alkylènes de vinyle de poly(éthylène glycol), dans lesquels R₁ = H ou CH₃ ; Z = -CH₂-O- ; R₂ = H ou alkyl en C₁-C₃₀ ; avec n = 2 à 200 ;
- les butylène d'oxyvinyle de poly(éthylène glycol), dans lesquels R₁ = H ou CH₃ ; Z = liaison covalente ; R₂ = H ou alkyl en C₁-C₃₀ ; avec n = 2 à 200 ;

5. Copolymère selon la revendication 4, ***caractérisé* en ce que** le monomère de formule (I) est choisi dans le groupe comprenant les (méth)acrylates de poly(éthylène glycol) et les (méth)acrylates de méthyl-poly(éthylène glycol), présentant une masse molaire comprise entre 80 et 8000 g/mol.

6. Copolymère selon l'une des revendications précédentes, ***caractérisé* en ce que** le monomère cationique est choisi dans le groupe comprenant l'acrylate de diméthylaminoéthyl (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC) et le chlorure de methacrylamido propyltrimethyl ammonium (MAPTAC).

7. Copolymère selon l'une des revendications précédentes, ***caractérisé* en ce que** le monomère non ionique est choisi dans le groupe comprenant l'acrylamide et le méthacrylamide, le N-isopropylacrylamide, le N,N-diméthylacrylamide, le N-méthylolacrylamide, la N-vinylformamide, le N-vinyl acetamide, la N-vinylpyridine, la N-vinylpyrrolidone, l'acryloyl morpholine (ACMO), et la diacétone acrylamide.

8. Copolymère selon la revendication 1, ***caractérisé* en ce que** n est un nombre entier compris entre 7 et 45.

9. Utilisation du copolymère objet de l'une des revendications précédentes, pour améliorer la formation de coacervats dans une formulation cosmétique ou détergente.

## Patentansprüche

1. Wasserlösliches Copolymer, umfassend bezogen auf das Gesamtgewicht des Copolymers:
- 1 bis 40 Gew.-% mindestens eines kationischen Monomers, dessen Kationizität ausschließlich aus der quaternären Ammoniumfunktion bzw. den quaternären Ammoniumfunktionen stammt;
- 59,99 bis 98 Gew.-% mindestens eines nicht-ionischen Monomers;
- 0,01 bis 10 Gew.-% mindestens eines Monomers der Formel (I); 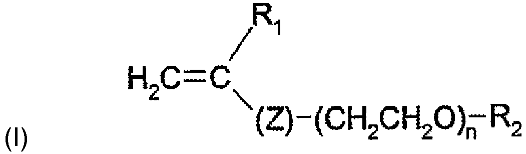 wobei:
- R₁ ein Wasserstoffatom oder ein Methylrest ist;
- Z für eine Funktion steht, ausgewählt aus der Gruppe umfassend eine kovalente Bindung, ein Sauerstoffatom, die zweiwertige Gruppe -CH₂- O-, -C(=O)-O-, und -C(=O)-NH-;
- n eine ganze Zahl im Bereich zwischen 2 und 200 ist;
- R₂ ein Wasserstoffatom oder ein gesättigter oder ungesättigter, gegebenenfalls aromatischer, geradkettiger, verzweigter oder cyclischer kohlenstoffhaltiger Rest ist, umfassend von 1 bis 30 Kohlenstoffatome und von 0 bis 4 Heteroatome, ausgewählt aus der Gruppe umfassend O, N, und S;
wobei das Copolymer eine kationische Ladungsdichte im Bereich zwischen 0,05 und 3 meq/g aufweist.

2. Copolymer nach Anspruch 1, ***dadurch gekennzeichnet,* dass** das Copolymer bezogen auf das Gesamtgewicht des Copolymers umfasst:
- 1 bis 30 Gew.-%, vorteilhafterweise zwischen 22,5 et 30 Gew.-% mindestens eines kationischen Monomers, dessen Kationizität ausschließlich aus der quaternären Ammoniumfunktion bzw. den quaternären Ammoniumfunktionen stammt;
- 60 bis 90 Gew.-%, vorteilhafterweise zwischen 60 und 80 Gew.-%, vorzugsweise zwischen 60 und 70 Gew.-% mindestens eines nicht-ionischen Monomers;
- 0,01 bis 10 Gew.-% mindestens eines Monomers der Formel (I);

3. Copolymer nach Anspruch 1, ***dadurch gekennzeichnet,* dass** das Copolymer eine kationische Ladungsdichte im Bereich zwischen 0,05 und 1,8 meq/g aufweist.

4. Copolymer nach einem der vorhergehenden Ansprüche, ***dadurch gekennzeichnet,* dass** das Monomer der Formel (I) ausgewählt ist aus der Gruppe umfassend:
- das Poly(ethylenglykol) (meth)acrylat, wobei R₁ = H oder CH₃; Z = C(=O)-O; R₂ = H; n = 2 bis 200;
- das Methyl-Poly(ethylenglykol) (meth)acrylat, wobei R₁ = H oder CH₃; Z = C(=O)-O-; R₂ = CH₃; n = 2 bis 200;
- die Alkyl-poly(ethylenglykol) (meth)acrylate, wobei R₁ = H oder CH₃; Z = C(=O)-O; R₂ = C₁-C₃₀-Alkyl, n = 2 bis 200;
- die Phenyl-poly(ethylenglykol) (meth)acrylate, wobei R₁ = H oder CH₃; Z = C(=O)-O; R₂ = Phenyl; n = 2 bis 200.
- die Poly(ethylenglykol)vinylakylene, wobei R₁ = H oder CH₃; Z = -CH₂-O-; R₂ = H oder C₁-C₃₀-Alkyl, mit n = 2 bis 200;
- die Poly(ethylenglykol)oxyvinylbutylene, wobei R₁ = H oder CH₃; Z = kovalente Bindung; R₂ = H oder C₁-C₃₀-Alkyl; mit n = 2 bis 200;

5. Copolymer nach Anspruch 4, ***dadurch gekennzeichnet,* dass** das Monomer der Formel (I) ausgewählt ist aus der Gruppe umfassend die Poly(ethylenglykol) (meth)acrylate und die Methyl-Poly(ethylenglykol) (meth)acrylate, mit einer Molmasse im Bereich zwischen 80 und 8000 g/mol.

6. Copolymer nach einem der vorhergehenden Ansprüche, ***dadurch gekennzeichnet,* dass** das kationische Monomer ausgewählt ist aus der Gruppe umfassend das quaternisierte Dimethylaminoethylacrylat (DMAEA), das quaternisierte Dimethylaminoethylmethacrylat (DMAEMA), das Dimethyldiallylammoniumchlorid (DADMAC), das Acrylamidpropyltrimethylammoniumchlorid (APTAC) und das Methacrylamidpropyltrimethylammoniumchlorid (MAPTAC).

7. Copolymer nach einem der vorhergehenden Ansprüche, ***dadurch gekennzeichnet,* dass** das nicht-ionische Monomer ausgewählt ist aus der Gruppe umfassend das Acrylamid und das Methacrylamid, das N-Isopropylacrylamid, das N,N-Dimethylacrylamid, das N-Methylolacrylamid, das N-Vinylformamid, das N-Vinylacetamid, das N-Vinylpyridin, das N-Vinylpyrrolidon, das Acryloylmorpholin (ACMO) und das Diacetonacrylamid.

8. Copolymer nach Anspruch 1, ***dadurch gekennzeichnet,* dass** n eine ganze Zahl im Bereich zwischen 7 und 45 ist.

9. Verwendung des Copolymers nach einem der vorhergehenden Ansprüche, zum Verbessern der Bildung von Koazervaten in einer kosmetischen oder reinigenden Formulierung.

## Claims

1. A water-soluble copolymer comprising, by mass relative to the total mass of the copolymer:
- 1 to 40% of at least one cationic monomer whose cationicity comes exclusively from one or more quaternary ammonium functional groups;
- 59.99 to 98% of at least one nonionic monomer;
- 0.01 to 10% of at least one monomer of formula (I); 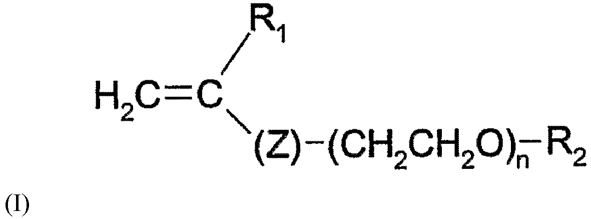 in which:
- R₁ is a hydrogen atom or a methyl radical;
- Z is a functional group chosen from the group comprising a covalent bond, an oxygen atom, the divalent group -CH₂ - O-, -C(=O)-O-, and -C(=O)-NH-;
- n is an integer between 2 and 200;
- R₂ is a hydrogen atom or a carbon-containing radical which is saturated or unsaturated, optionally aromatic, linear, branched or cyclic, comprising 1 to 30 carbon atoms, and from 0 to 4 heteroatoms chosen from the group comprising O, N and S;
the copolymer having a cationic charge density between 0.05 and 3 meq/g.

2. Copolymer as claimed in claim 1, wherein the copolymer comprises by mass relative to the total mass of the copolymer:
- 1 to 30%, advantageously between 22.5 and 30% of at least one cationic monomer whose cationicity comes exclusively from quaternary ammonium functional groups;
- 60 to 90% advantageously between 60 and 80% preferably between 60 and 70% of at least one nonionic monomer;
- 0.01 to 10% of at least one monomer of formula (I).

3. Copolymer as claimed in claim 1, wherein the copolymer has a cationic charge density between 0.05 and 1.8 meq/g.

4. Copolymer as claimed in one of the preceding claims, wherein the monomer of formula (I) is chosen from the group comprising:
- poly(ethylene glycol) (meth)acrylate in which R₁ = H or CH₃; Z = C(=O)-O-; R₂ = H; n = 2 to 200;
- methyl-poly(ethylene glycol) (meth)acrylate, in which R₁ = H or CH₃; Z = C(=O)-O-; R₂ = CH₃; n = 2 to 200;
- alkyl-poly(ethylene glycol) (meth)acrylates in which R₁ = H or CH₃; Z = C(=O)-O-; R₂ = C₁-C₃₀ alkyl; n = 2 to 200;
- phenyl-poly(ethylene glycol) (meth)acrylates, in which R₁ = H or CH₃; Z = C(=O)-O-; R₂ = phenyl; n = 2 to 200;
- poly(ethylene glycol) vinyl alkylenes, in which R₁ = H or CH₃; Z = -CH2-O-; R₂ = H or C₁-C₃₀ alkyl; with n = 2 to 200;
- poly(ethylene glycol) oxyvinyl butylenes, in which R₁ = H or CH₃; Z = covalent bond; R₂ = H or C₁-C₃₀ alkyl; with n = 2 to 200.

5. Copolymer as claimed in claim 4, wherein the monomer of formula (I) is chosen from the group comprising poly(ethylene glycol) (meth)acrylates and methyl-poly(ethylene glycol) (meth)acrylates, having a molar mass between 80 and 8000 g/mol.

6. Copolymer as claimed in one of the preceding claims, wherein the cationic monomer is chosen from the group comprising quaternized dimethylaminoethyl acrylate (ADAME), quaternized dimethylaminoethyl methacrylate (MADAME), dimethyldiallylammonium chloride (DADMAC), acrylamidopropyltrimethylammonium chloride (APTAC) and methacrylamidopropyltrimethylammonium chloride (MAPTAC).

7. Copolymer as claimed in one of the preceding claims, wherein the nonionic monomer is chosen from the group comprising acrylamide and methacrylamide, N-isopropylacrylamide, N,N-dimethylacrylamide, N-methylolacrylamide, N-vinylformamide, N-vinylacetamide, N-vinylpyridine, N-vinylpyrrolidone, acryloylmorpholine (ACMO) and diacetone acrylamide.

8. Copolymer according to claim 1, wherein n is an integer between 7 and 45.

9. The use of the copolymer which is the subject of one of the preceding claims, in order to improve the coacervat formation in a cosmetic or detergent formulation.
